# FASCICULE DE BREVET EUROPEEN

(11) **EP 3 883 493 B1**
(45) Date de publication et mention de la délivrance du brevet: **02.08.2023**
(21) Numéro de dépôt: 19839344.9
(22) Date de dépôt: 25.11.2019
(51) Int. Cl.: A61C 7/00, A61C 13/00, G16H 50/50

(54) **PROCÉDÉ D'ANIMATION DE MODÈLES DES ARCADES MANDIBULAIRE ET MAXILLAIRE D'UN PATIENT DANS UNE RELATION INTERMAXILLAIRE CORRIGÉE**
VERFAHREN ZUR ANIMATION VON MODELLEN VON UNTERKIEFER UND OBERKIEFER EINES PATIENTEN IN KORRIGIERTER INTERMAXILLÄRER BEZIEHUNG
METHOD FOR ANIMATING MODELS OF THE MANDIBULAR AND MAXILLARY ARCHES OF A PATIENT IN A CORRECTED INTERMAXILLARY RELATIONSHIP

(30) Priorité: 23.11.2018 FR 1871744
(43) Date de publication de la demande: 29.09.2021
(73) Titulaire: Modjaw, 69100 Villeurbanne (FR)
(72) Inventeur: JAISSON, Maxime, 73800 Les Marches (FR)
(74) Mandataire: Regimbeau
(86) Numéro de dépôt international: PCT/FR2019/052798
(87) Numéro de publication internationale: WO 2020/104760

(56) Documents cités:
- US-A1- 2002 048 741
- US-A1- 2009 068 617
- US-A1- 2011 191 081
- US-A1- 2018 005 377

## Description

### DOMAINE DE L'INVENTION

La présente invention se situe dans le domaine de la dentisterie. Elle concerne plus précisément un procédé d'animation de modèles de l'arcade mandibulaire et de l'arcade maxillaire d'un patient dans une relation intermaxillaire corrigée.

### ETAT DE LA TECHNIQUE

La dimension verticale d'occlusion (souvent désignée par l'acronyme DVO) est une grandeur importante de la face d'un individu, tant sur le plan esthétique (harmonie des proportions) que sur le plan fonctionnel (confort associé à la fonction temporo-mandibulaire).

La dimension verticale d'occlusion est généralement définie comme étant la hauteur de l'étage inférieur de la face, ou, plus simplement, la distance qui sépare le point sous-nasal du gnathion, pendant la phase d'occlusion. De manière conventionnelle, le point sous-nasal est défini comme étant l'épine nasale antérieure, tandis que le gnathion est le point le plus antérieur de la partie la plus déclive de la protubérance mentonnière.

En dentisterie, il est parfois nécessaire d'augmenter la dimension verticale d'occlusion d'un patient. Cette augmentation peut être motivée par le fait que le patient a perdu sa dimension verticale d'occlusion originelle, par exemple du fait d'une érosion dentaire d'origine chimique ou mécanique. Une autre indication d'une augmentation de la dimension verticale d'occlusion peut être la mise en oeuvre d'un traitement thérapeutique qui nécessite suffisamment d'espace pour placer des prothèses ou des appareils d'orthodontie.

L'augmentation de la dimension verticale d'occlusion nécessite de modifier la position de la mandibule par rapport au maxillaire (également appelée relation intermaxillaire (RIM)), ce qui engendre une modification de la zone de confrontation des arcades dentaires. Selon l'amplitude de l'augmentation, la mandibule peut être déplacée uniquement dans une direction verticale et éventuellement dans une direction antéro-postérieure.

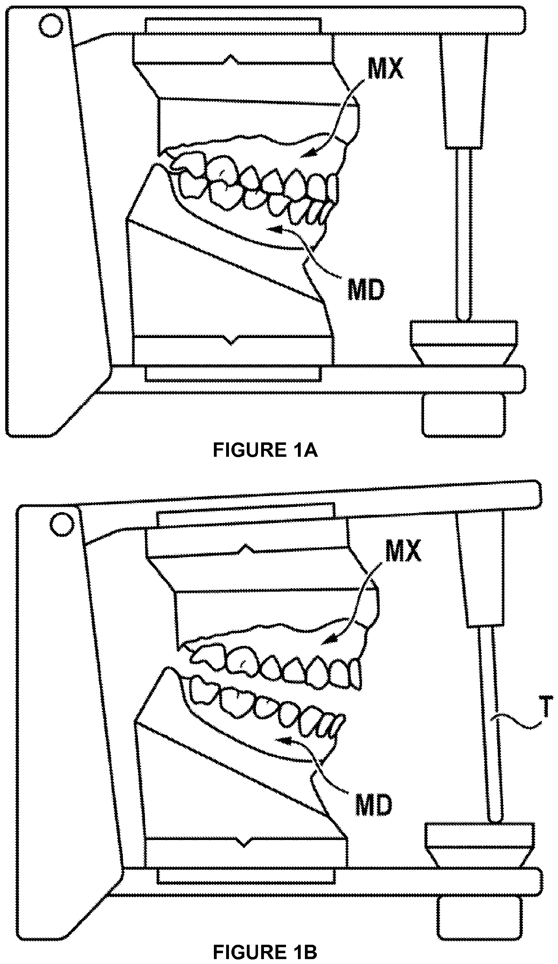

Pour s'assurer que cette nouvelle relation intermaxillaire sera confortable et pertinente pour le patient, le praticien doit simuler les mouvements appliqués aux arcades dentaires, en utilisant un articulateur mécanique ou virtuel utilisant des modèles physiques ou numériques tridimensionnels de l'arcade mandibulaire et de l'arcade maxillaire. Le choix de la nouvelle position mandibulaire est effectué par un enregistrement en bouche sur une feuille de cire ou par injection d'un matériau à prise chimique. Lorsque le déplacement de la mandibule est uniquement vertical, la modification peut être effectuée directement sur l'articulateur, en déplaçant la tige incisive T de sorte à espacer le modèle de l'arcade mandibulaire MD du modèle de l'arcade maxillaire MX d'une distance égale à l'augmentation souhaitée pour la dimension verticale d'occlusion souhaitée (cf. figures 1A et 1B). Cependant, il est recommandé d'utiliser un arc facial afin de situer les modèles par rapport à l'axe de rotation de la mandibule pour que l'espacement des arcades soit le plus proche possible de la réalité.

Toutefois, de tels articulateurs fournissent des simulations imprécises dans la mesure où la cinématique appliquée est approximée et ne correspond pas à la cinématique mandibulaire réelle (également appelée fonction réelle) du patient.

US 2011/191081 A1, US 2018/005377 A1, US 2009/068617 A1 et US 2002/048741 A1 divulguent des procédé d'animation de modèles des arcades mandibulaire et maxillaire d'un patient.

### EXPOSE DE L'INVENTION

Un but de l'invention est de remédier aux inconvénients des techniques connues et de proposer un procédé d'animation de modèles des arcades mandibulaire et maxillaire d'un patient dans une relation intermaxillaire corrigée.

A cet effet, l'invention propose un procédé d'animation de modèles des arcades mandibulaire et maxillaire d'un patient dans une relation intermaxillaire corrigée, comprenant :
- la fourniture de modèles numériques tridimensionnels des arcades mandibulaire et maxillaire du patient,
- la fourniture d'un enregistrement de la cinématique mandibulaire du patient, ledit enregistrement comprenant une pluralité de positions relatives de la mandibule du patient par rapport au maxillaire,
- la sélection, parmi lesdites positions, d'une position de référence de la mandibule par rapport au maxillaire du patient,
- la détermination d'une position cible de la mandibule par rapport au maxillaire, ladite position cible définissant une relation intermaxillaire corrigée du patient,
- la détermination d'une transformation rigide entre la position de référence (X) et la position cible,
- l'application de ladite transformation rigide (i) à l'enregistrement de la cinématique mandibulaire du patient pour animer les modèles numériques tridimensionnels des arcades mandibulaire et maxillaire avec l'enregistrement résultant de l'application de ladite transformation rigide, ou (ii) au modèle numérique tridimensionnel de la mandibule, l'enregistrement de la cinématique mandibulaire fourni étant appliqué aux modèles numériques tridimensionnels de la mandibule et du maxillaire résultant de l'application de ladite transformation rigide pour animer lesdits modèles.

La position de référence étant présente dans l'enregistrement de la cinématique mandibulaire, les données de la cinématique mandibulaire sont attachées à cette position de référence. La présente invention permet de transposer lesdites données à une autre position, dite position cible.

Selon un mode de réalisation, la position de référence est la position occlusale de référence du patient avant la correction de la relation intermaxillaire.

Selon un mode de réalisation, l'enregistrement de la cinématique mandibulaire du patient décrit la trajectoire de marqueurs solidaires de la mandibule, et la transformation rigide est une transformation permettant de passer de la position des marqueurs dans la position de référence à la position des marqueurs dans la position cible.

La position cible peut être choisie par l'utilisateur parmi l'ensemble des positions relatives de la mandibule par rapport au maxillaire dans l'enregistrement de la cinématique mandibulaire.

De manière alternative, le procédé comprend la présélection automatique d'un ensemble de positions relatives de la mandibule par rapport au maxillaire, ledit ensemble de positions étant choisi de sorte que chaque position dudit ensemble définisse une dimension verticale d'occlusion dans une gamme déterminée.

De manière avantageuse, ladite présélection peut être réalisée à partir de critères cliniques préalablement fournis.

Dans d'autres modes de réalisation, la position cible ne fait pas partie de l'ensemble des positions présentes dans l'enregistrement de la cinématique mandibulaire, mais peut être simulée. A cet effet, le procédé comprend avantageusement la détermination d'un axe de rotation de la mandibule et la création de positions virtuelles de la mandibule sur le trajet de la rotation dans le sens d'une augmentation ou d'une réduction de la dimension verticale d'occlusion, la position cible étant choisie parmi lesdites positions virtuelles.

Dans certaines formes d'exécution, la position cible est choisie de sorte que la dimension verticale d'occlusion (DVO) soit augmentée d'une valeur inférieure à un seuil choisi pour ne pas provoquer de déplacement de l'axe de rotation des condyles mandibulaires par rapport à la fosse temporale du patient.

De préférence, la position cible est choisie de sorte que la dimension verticale d'occlusion définie au niveau du dentalé soit augmentée de moins de 20 mm par rapport à la dimension verticale d'occlusion définie par la position occlusale de référence du patient.

Dans d'autres formes d'exécution, la position cible est choisie de sorte que la dimension verticale d'occlusion (DVO) soit diminuée.

Dans l'animation des modèles des arcades mandibulaire et maxillaire, la position occlusale de référence de la mandibule par rapport au maxillaire dans la relation intermaxillaire corrigée devient la position cible.

### BREVE DESCRIPTION DES FIGURES

D'autres caractéristiques et avantages de l'invention ressortiront de la description détaillée qui va suivre, en référence aux dessins annexés sur lesquels :
- la figure 1A illustre un articulateur physiologique avant augmentation de la dimension verticale d'occlusion ;
- la figure 1B illustre l'articulateur physiologique de la figure 1A après augmentation de la dimension verticale d'occlusion ;
- la figure 2A illustre l'enregistrement de la cinématique mandibulaire du patient respectivement avant la correction de la relation intermaxillaire ;
- la figure 2B illustre le choix de la frame définissant la position cible de la mandibule dans la relation intermaxillaire corrigée ;
- la figure 2C illustre l'application de l'enregistrement de la cinématique mandibulaire du patient après la correction de la relation intermaxillaire ;
- la figure 3 présente de manière schématique les déplacements de la mandibule en fonction de l'ouverture occasionnée par l'augmentation de la dimension verticale d'occlusion ;
- la figure 4 illustre différents motifs de mastication enregistrés au niveau du dentalé pour des cycles fonctionnels et dysfonctionnels.

### DESCRIPTION DETAILLEE DE MODES DE REALISATION DE L'INVENTION

Le procédé décrit ci-après est mis en oeuvre par ordinateur.

Le système permettant la mise en oeuvre du procédé comprend :
- un ordinateur comprenant un processeur pour recevoir des données préalablement obtenues, notamment des modèles numériques des arcades mandibulaire et maxillaire du patient et un enregistrement de la cinématique mandibulaire dudit patient, et pour faire fonctionner des algorithmes d'animation et de modélisation, et
- un écran pour afficher les différentes étapes mises en oeuvre au cours du procédé, avec une interface utilisateur permettant au praticien d'entrer des paramètres du traitement et de visualiser la cinématique mandibulaire avant et après correction.

### Obtention des modèles numériques tridimensionnels des arcades mandibulaire et maxillaire

Préalablement à la mise en oeuvre du procédé, les arcades dentaires ont été scannées dans une relation d'engrènement connue et reproductible. Un outil utilisable à cet effet est une caméra d'empreinte optique intrabuccale. Une telle caméra est utilisée pour scanner une arcade puis l'autre, ainsi qu'une empreinte vestibulaire (sur le côté) des dents en engrènement pour connaître la position d'une arcade par rapport à l'autre. Cette opération est connue en elle-même et ne fait pas en tant que telle partie de l'invention.

Une autre méthode peut consister à utiliser un scanner de table. Celui-ci scanne les modèles en plâtre issus d'empreintes physicochimiques l'un après l'autre puis en position d'engrènement.

D'autres méthodes sont également envisageables, tels qu'un scannage radiographique par cone beam d'un modèle en plâtre ou d'une empreinte en silicone des arcades dentaires.

L'invention peut en effet être mise en oeuvre avec tout modèle numérique tridimensionnel des arcades dentaires généré par les techniques de numérisation disponibles sur le marché. Le résultat de ces numérisations est un maillage surfacique de l'arcade maxillaire et de l'arcade mandibulaire. Chaque maillage est défini dans un repère orthonormé du dispositif de numérisation.

### Enregistrement de la cinématique mandibulaire

L'enregistrement de la cinématique mandibulaire a pour but de permettre de connaître la façon dont se déplace la mandibule dans l'espace, et d'utiliser cette cinématique pour animer les modèles tridimensionnels des arcades dentaires en vue de guider la conception d'une prothèse dentaire ou d'un autre dispositif de correction (appareil d'orthodontie, gouttière, ...).

Un mode de réalisation de cet enregistrement, ainsi que du recalage des modèles des arcades dentaires par rapport à des plans ou axes de référence du patient a déjà été décrit dans le document WO 2013/030511 et peut être mis en oeuvre dans la présente invention, l'invention n'étant toutefois pas limitée aux techniques décrites dans ce document.

D'une manière générale, l'enregistrement de la cinématique mandibulaire est mis en oeuvre en équipant le patient d'au moins un marqueur solidaire du front du patient (ou tout autre emplacement de la face solidaire du maxillaire) et d'au moins un marqueur solidaire de l'arcade mandibulaire (par fixation sur la mandibule par l'intermédiaire d'un support ou par fixation directe sur les dents), et en repérant et enregistrant les déplacements desdits marqueurs au moyen d'une caméra lors de mouvements mandibulaires du patient.

Selon la technologie de la caméra, les marqueurs peuvent être des diodes, des mires noires et blanches ou en couleur ou encore des sphères, des pastilles ou autres objets réfléchissants. Le déplacement des marqueurs de la mandibule est suivi par la caméra et cela par rapport aux marqueurs du front. Une transformation rigide permet de déduire le mouvement du modèle de l'arcade mandibulaire par rapport au modèle de l'arcade maxillaire.

Il est possible de s'affranchir d'un marqueur frontal en mettant en oeuvre une technique de reconnaissance faciale telle que décrite dans le document WO 2016/062962. Cette technique permet en effet de déterminer, à partir d'images stéréoscopiques, des points et plans de référence du patient, par rapport auxquels on suit le déplacement des marqueurs de la mandibule.

L'invention n'est pas limitée à une technique particulière d'acquisition de la cinématique mandibulaire. Par exemple, le déplacement de la mandibule par rapport au maxillaire peut être enregistré au moyen d'accéléromètres ou de centrales inertielles solidaires des mâchoires.

### Détermination de la position de référence et de la position cible

L'enregistrement de la cinématique mandibulaire comprend une pluralité de positions successives de la mandibule par rapport au maxillaire au cours de mouvements réalisés par le patient (par exemple la mastication).

L'enregistrement se présente sous la forme d'un fichier numérique comprenant une pluralité de frames correspondant chacune à une position relative de la mandibule et du maxillaire.

Ce fichier numérique comprend en particulier une frame correspondant à une position de référence du patient (par exemple, mais de manière non limitative, la position occlusale de référence). C'est à cette position de référence que sont attachées les données de la cinématique mandibulaire.

Dans certains modes de réalisation, ce fichier numérique peut comprendre en outre une frame correspondant à une position cible destinée à devenir la nouvelle position de référence du patient après mise en oeuvre de la correction.

Dans d'autres modes de réalisation, notamment si aucune des frames de l'enregistrement de la cinématique mandibulaire ne correspond à une position cible convenable pour le praticien, le praticien peut simuler la position cible. A cet effet, à l'aide de l'enregistrement de la cinématique mandibulaire, un algorithme peut déterminer l'axe d'articulation de la mandibule par rapport au maxillaire. Cet axe passe typiquement par les condyles. Le praticien peut alors simuler une rotation du modèle numérique de l'arcade mandibulaire autour de cet axe pour déterminer une position préférée de la mandibule par rapport au maxillaire. C'est cette position préférée qui est choisie comme position cible. Une ou plusieurs frames peuvent être créées pour représenter des positions virtuelles de la mandibule sur le trajet de la rotation dans le sens d'une ouverture ou d'une réduction de la dimension verticale d'occlusion. En pratique, ces frames supplémentaires sont créées par application d'une transformation rigide de type rotation seule autour de l'axe de rotation des condyles préalablement déterminé, sur les modèles tridimensionnels des frames enregistrées. Ces frames supplémentaires s'ajoutent aux frames de la cinématique mandibulaire réellement enregistrée. La position cible peut alors être une seconde frame choisie par le praticien parmi la ou les frame(s) supplémentaire(s).

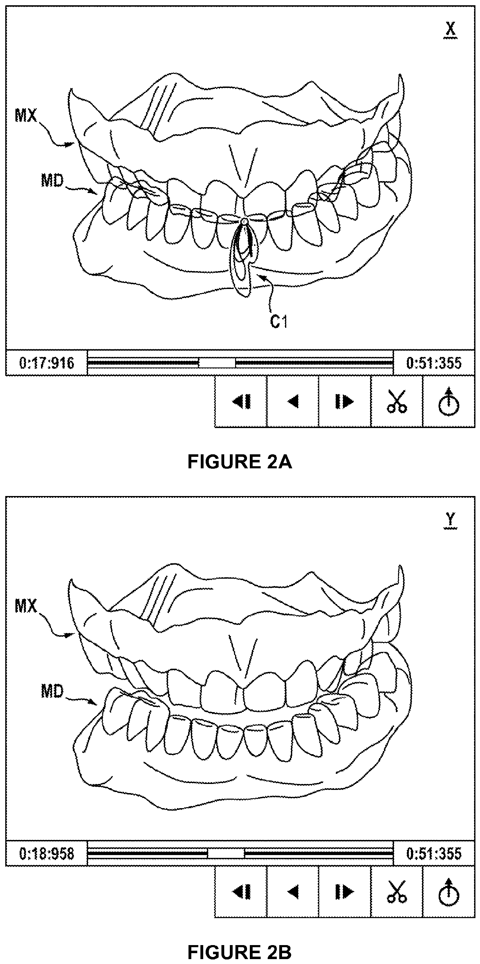

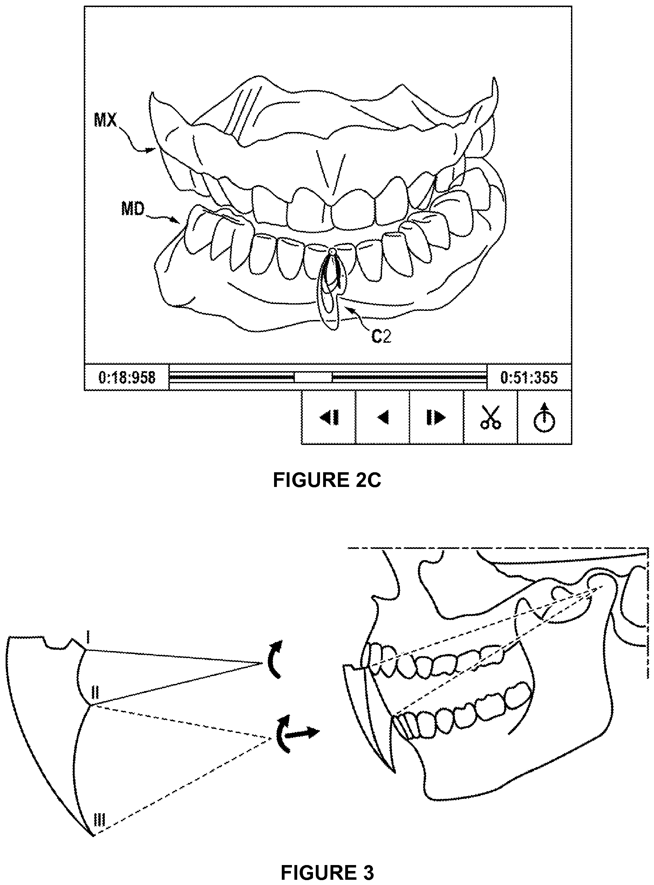

Les figures 2A à 2C illustrent l'enregistrement de la cinématique mandibulaire du patient respectivement avant (figure 2A) et après (figure 2C) la correction de la relation intermaxillaire.

L'utilisateur peut afficher sur l'écran ledit enregistrement et le jouer autant de fois que nécessaire. A cet effet, il dispose d'une interface utilisateur comprenant une zone d'affichage pour visualiser les mouvements de l'arcade mandibulaire MD et de l'arcade maxillaire MX et des boutons pour passer d'une frame à une autre et pour exporter une frame particulière.

L'utilisateur sélectionne une première frame X qui correspond à la position de référence du patient.

Selon une forme d'exécution, la position de référence est par convention celle de la première frame de l'enregistrement de la cinématique mandibulaire. En général, le patient est supposé commencer l'enregistrement avec les dents serrées, ce qui correspond à la position d'engrènement des modèles numériques des arcades, dite position occlusale de référence.

Selon une autre forme d'exécution, le praticien peut être amené à placer un dispositif interocclusal (par exemple une gouttière) au cours de l'enregistrement de la cinématique mandibulaire pour guider les mouvements mandibulaires et trouver une position particulière de la mandibule par rapport au maxillaire, autre que la position d'engrènement initiale.

L'homme du métier pourra éventuellement définir d'autres critères pour définir la position de référence.

La figure 2A illustre par exemple l'arcade mandibulaire MD dans ladite position de référence. La cinématique mandibulaire est représentée sous la forme de la courbe C1 qui illustre le motif de mastication du patient au niveau du dentalé.

L'utilisateur sélectionne par ailleurs une seconde frame Y qui correspond à la position cible destinée à devenir la nouvelle position de référence du patient (cf. figure 2B). Cette position cible est associée à une relation intermaxillaire corrigée, correspondant par exemple à une dimension verticale d'occlusion augmentée ou diminuée par rapport à la dimension verticale d'occlusion initiale du patient.

L'utilisateur peut effectuer la sélection de la seconde frame Y à partir de l'ensemble des frames constituant l'enregistrement. Il fait pour cela appel à son expertise technique afin de déterminer l'amplitude de l'augmentation ou diminution de la dimension verticale d'occlusion adaptée pour le patient. Cette sélection est effectuée sur le trajet d'ouverture-fermeture en choisissant un mouvement répétable. Ce mouvement peut être généré par le patient lui-même ou guidé par le praticien qui manipule la mandibule du patient pour ressentir le mouvement axial terminal et vérifier que le retour à l'occlusion se fait toujours au même endroit de confrontation interarcade.

De manière alternative, un algorithme de traitement de l'enregistrement de la cinématique mandibulaire peut effectuer une présélection d'un ensemble de frames, de sorte à limiter le choix de l'utilisateur à un nombre restreint de frames correspondant chacune à une dimension verticale d'occlusion dans une gamme déterminée. On peut utiliser pour cela des algorithmes de type « intelligence artificielle ». Cette présélection est avantageusement basée sur des critères cliniques préalablement entrés dans l'algorithme.

On pourra ainsi se référer à l'article de Jean-François Lasserre [1], qui fournit des règles à respecter dans le cadre d'une augmentation de la dimension verticale d'occlusion. Une de ces règles est dite « règle des 1/3 » ; elle prend en compte le fait que, sur un articulateur physiologique, pour une même augmentation de DVO de l'étage inférieur de la face, l'ouverture entre la tige incisive et la table incisive est trois fois l'ouverture entre les deuxièmes molaires, et l'ouverture entre les incisives centrales est deux fois celle entre les deuxièmes molaires. Une autre règle est celle dite des « vides fonctionnels » ou « 1+1=3 » ; elle traduit le fait que, lors de la réalisation d'overlays antagonistes, il faut tenir compte, en plus de l'épaisseur desdits overlays, des vides occlusaux fonctionnels, dont l'épaisseur est de l'ordre de 1 mm pour des overlays de 1 mm d'épaisseur chacun.

Sur la base de ces règles, le logiciel peut proposer, lorsque le praticien cherche à déterminer une nouvelle position mandibulaire, une situation de la mandibule telle que les premières molaires se trouvent espacées de 3 mm, qui est l'espace minimal pour la réalisation de prothèses résistantes. Sur le trajet de rotation, le logiciel arrête donc la mandibule une fois que cet espace est atteint.

De manière alternative, des tracés céphalométriques sur une radiographie de profil permettent, grâce au calcul de certains angles, de déterminer la position cible de la mandibule dans un plan sagittal. La radiographie de profil importée dans le logiciel est superposée aux modèles 3D. Les résultats de l'analyse céphalométrique proposent une position de la mandibule sur le chemin des mouvements préenregistrés.

Selon une autre forme d'exécution, le mouvement de déglutition permet, dans certaines situations cliniques, de choisir la dimension verticale d'occlusion, notamment pour un patient édenté. En effet, la mandibule marque un temps d'arrêt sur une position la plus haute, qui est une position que le logiciel peut proposer au praticien.

Comme indiqué plus haut, la position cible peut ne pas être choisie comme une frame de l'enregistrement de la cinématique mandibulaire, mais peut être simulée.

En général, il est préférable de limiter l'augmentation de la dimension verticale d'occlusion en-dessous d'un certain seuil. En effet, le mouvement mandibulaire est guidé par les dents, l'articulation temporo-mandibulaire et l'activité neuromusculaire du patient. Comme illustré sur la figure 3, tant que l'augmentation de la dimension verticale d'occlusion est inférieure audit seuil (ouverture de I à II), l'axe de rotation des condyles reste dans la fosse temporale et les muscles de l'articulation restent dans leur domaine de résilience ; dans ce cas, seules des modifications de la dentition sont susceptibles de modifier le mouvement mandibulaire. En revanche, si l'augmentation de la dimension verticale d'occlusion est supérieure audit seuil (ouverture de II à III), il se produit également une translation de la mandibule dans un plan horizontal, une suppression du guidage dentaire et une modification de l'activité musculaire ; dans ce cas, l'enveloppe du mouvement mandibulaire est modifiée, ce que l'on souhaite éviter.

En pratique, on choisit de préférence la position cible de sorte que la dimension verticale d'occlusion définie au niveau du dentalé soit augmentée de moins de 20 mm par rapport à la dimension verticale d'occlusion définie par la position occlusale de référence initiale du patient, afin de ne pas déplacer l'axe de rotation des condyles.

Dans certains cas, le praticien peut être au contraire confronté à la nécessité de diminuer la dimension verticale d'occlusion du patient. Tel est le cas par exemple lors de la correction des prématurités, c'est-à-dire des contacts qui, pour des raisons pathologiques, se produisent de manière prématurée lors de la rotation de la mandibule. Une prématurité peut par exemple être causée par une dent trop proéminente par rapport à l'arcade à laquelle elle appartient. Pour autant, la cinématique mandibulaire peut être correcte. La position cible peut être déterminée en simulant une abrasion de la dent proéminente et en simulant la fermeture des mâchoires qui en résulte.

Un autre exemple concerne un cas où le patient est équipé d'une gouttière qui évite ou tout au moins réduit les mouvements erratiques de la mandibule dans le cadre d'un traitement de reconditionnement neuromusculaire ; une telle gouttière engendrant une surépaisseur au niveau de l'arcade qu'elle recouvre, elle est susceptible d'augmenter la distance verticale d'occlusion par rapport à la situation post-traitement où le patient ne porte pas la gouttière. Dans ce cas, la cinématique mandibulaire générée par le port de la gouttière est d'intérêt pour le praticien.

Il existe différentes manières de procéder dans le cas de l'utilisation d'une gouttière.

Dans un mode de réalisation, les arcades dentaires sont scannées avec la gouttière en place sur la mandibule ou le maxillaire, et en position d'occlusion sur cette gouttière. La position de référence (matérialisée par la première frame X) correspond à cette situation. La position cible peut exister dans l'enregistrement de la cinématique mandibulaire ou peut être simulée. La position cible peut exister si l'enregistrement de la cinématique mandibulaire commence avec la gouttière en place puis que le praticien enlève pendant l'enregistrement la gouttière de la bouche du patient, celui-ci venant se placer ou étant guidé dans la position cible, matérialisée par une seconde frame Y. La position cible peut être simulée en étant choisie sur le trajet simulé de la mandibule lors de la rotation autour de son axe.

Dans un autre mode de réalisation, les arcades dentaires sont scannées sans la gouttière et en position d'engrènement dentaire. Au démarrage de l'enregistrement de la cinématique mandibulaire, la première frame est la position des arcades en occlusion, telles que scannées, puis, pendant ce même enregistrement, la gouttière est mise en place sur l'une des deux arcades et des exercices de déplacement de la mandibule sont effectués. La position de référence correspond à la frame au moment où la gouttière est en place (la cinématique à transposer étant celle lorsque que le patient est au contact de la gouttière). Le praticien la valide informatiquement comme telle et sélectionne aussi la partie de la cinématique mandibulaire attachée à cette nouvelle position de référence qu'il voudra transposer à la position cible. A l'écran on voit alors les deux modèles d'arcades dentaires se déplacer avec un espace entre eux. Ensuite la position cible est choisie comme une frame de l'enregistrement ou simulée. Si la position cible est choisie, elle peut être la première frame de l'enregistrement ou une autre position correspondant à une autre frame de la partie de l'enregistrement dans laquelle la gouttière n'est pas en place. La simulation de la position cible a été décrite plus haut.

### Détermination de la transformation rigide

Le choix des frames X et Y ayant été effectué, un algorithme calcule une transformation rigide de la frame X à la frame Y.

Différentes méthodes peuvent être employées pour calculer cette transformation rigide. Selon un mode de réalisation préféré, ce calcul est basé sur les trajectoires des marqueurs solidaires de la mandibule et du maxillaire lors de l'enregistrement de la cinématique mandibulaire. La transformation rigide est la transformation qui permet de passer de la position des marqueurs dans la frame X à la position desdits marqueurs dans la frame Y. Par exemple, si la position de MD par rapport à MX dans la frame X (respectivement la frame Y) est représentée par la matrice Tx (respectivement Ty), alors la transformation rigide de MD de la frame X à la frame Y est représentée par la matrice (Ty)⁻¹*Tx.

Naturellement, l'homme du métier pourrait utiliser d'autres méthodes de calcul de la transformation rigide en fonction de l'enregistrement dont il dispose.

### Animation des modèles des arcades

En référence à la figure 2C, un algorithme applique à la cinématique mandibulaire enregistrée la transformation rigide préalablement calculée (courbe C2). Les modèles des arcades mandibulaire et maxillaire sont alors animés avec la cinématique C2.

De manière alternative, un algorithme applique la transformation rigide au modèle de l'arcade mandibulaire et anime les modèles des arcades ayant subi ladite transformation rigide avec la cinématique mandibulaire initialement enregistrée du patient.

L'homme du métier pourra choisir l'une ou l'autre de ces méthodes d'animation en fonction des éventuelles contraintes informatiques et mathématiques auxquelles il est confronté pour mettre en oeuvre le procédé.

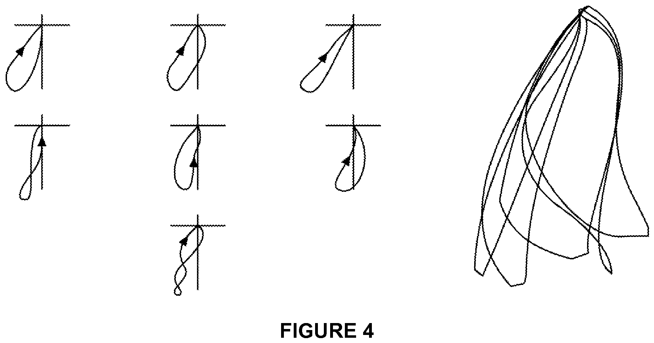

De manière avantageuse, le praticien vérifie si la cinématique mandibulaire du patient est fonctionnelle ou dysfonctionnelle avant de mettre en oeuvre la suite du procédé et notamment de transposer cette cinématique à la relation intermaxillaire corrigée. A cet effet, il peut tracer un motif de mastication et comparer ce motif avec des types de motifs identifiés comme fonctionnels ou dysfonctionnels. La figure 4 illustre ainsi, dans sa partie gauche, des motifs théoriques de mastication au niveau du dentalé pour des cycles fonctionnels (les trois cycles du haut) et des cycles dysfonctionnels (les quatre cycles du bas). La partie droite de la figure 4 illustre un motif clinique, qui est considéré comme fonctionnel. Naturellement, l'homme du métier pourrait tracer un motif de mastication enregistré au niveau d'un autre point que le dentalé, par exemple les condyles. Le procédé décrit plus haut est mis en oeuvre uniquement si la cinématique mandibulaire du patient est fonctionnelle. Dans le cas où cette cinématique est dysfonctionnelle, le praticien se tournera vers d'autres outils, tels qu'un articulateur.

### Applications

Le procédé qui vient d'être décrit peut trouver application dans les domaines de l'orthodontie, de la pose de prothèses dentaires ou dans la chirurgie maxillo-faciale.

Par exemple, il existe des situations où les dents d'un patient peuvent être abrasées par des contraintes mécaniques (bruxisme) ou par érosion chimique due à la consommation d'aliments ou de boissons acides. Les dents peuvent être naturelles ou prothétiques. Cette destruction dentaire se fait lentement et la fonction masticatrice a le temps de sculpter les dents en gardant les guidages essentiels sur les tables occlusales, malgré leur destruction et la perte de dimension verticale. Pour traiter un tel patient, un examen complémentaire de la santé de l'appareil manducateur (muscles et articulations) est établi préalablement à la mise en oeuvre de la présente invention. Si le résultat est positif, le praticien aura la volonté de préserver la façon dont la mandibule se meut, mais il devra rétablir la dimension verticale d'occlusion. Avant la conception de la présente invention, la seule solution résidait dans la mise en oeuvre des travaux sur un articulateur ne préservant pas les mouvements réels du patient. Dans cette situation clinique, l'invention vise à transposer la même cinématique mandibulaire, confortable pour le patient, à une nouvelle position de l'arcade mandibulaire. L'espace nouveau créé sera comblé par de nouveaux artifices prothétiques dont la forme et la position ne perturberont pas la motilité mandibulaire.

Dans le cas où un traitement orthodontique est envisagé, la démarche clinique est sensiblement la même que celle décrite ci-dessus. Au lieu de combler l'espace par des prothèses, c'est le déplacement des dents et leur mise en engrènement qui aura lieu. Dans ce cas, la mise en occlusion se fera en respectant également les vrais mouvements du patient.

Aussi en orthodontie il sera possible d'exporter la position avec la cinématique qui lui est accrochée après avoir fermé la dimension verticale d'occlusion suite à la correction d'une prématurité gênant le chemin de fermeture. Cette relation intermaxillaire corrigée avec les mouvements associés sera utile pour planifier le traitement orthodontique et préparer les artifices pour effectuer le traitement de façon plus prédictible.

### REFERENCES

WO 2013/030511
WO 2016/062962

[1] Jean-François Lasserre, « Comprendre l'augmentation de DVO dans les approches minimales invasives des traitements de l'usure et des anomalies de l'émail », Quintessence International

## Revendications

1. Procédé d'animation de modèles des arcades mandibulaire et maxillaire d'un patient dans une relation intermaxillaire corrigée, ledit procédé étant mis en oeuvre par ordinateur et comprenant :
- la réception de modèles numériques tridimensionnels (MD, MX) des arcades mandibulaire et maxillaire du patient,
- la réception d'un enregistrement de la cinématique mandibulaire (C1) du patient, ledit enregistrement comprenant une pluralité de positions relatives de la mandibule du patient par rapport au maxillaire,
- la sélection, parmi lesdites positions, d'une position de référence (X) de la mandibule par rapport au maxillaire du patient, et
- la détermination d'une position cible (Y) de la mandibule par rapport au maxillaire, ladite position cible définissant une relation intermaxillaire corrigée du patient,
- la détermination d'une transformation rigide entre la position de référence (X) et la position cible (Y),
- l'application de ladite transformation rigide (i) à l'enregistrement de la cinématique mandibulaire du patient pour animer les modèles numériques tridimensionnels des arcades mandibulaire et maxillaire, ou (ii) au modèle numérique tridimensionnel de la mandibule, l'enregistrement de la cinématique mandibulaire fourni étant appliqué aux modèles numériques tridimensionnels de la mandibule et du maxillaire pour animer lesdits modèles.

2. Procédé selon la revendication 1, dans lequel l'enregistrement de la cinématique mandibulaire du patient décrit la trajectoire de marqueurs solidaires de la mandibule, et la transformation rigide est une transformation permettant de passer de la position des marqueurs dans la position de référence (X) à la position des marqueurs dans la position cible (Y).

3. Procédé selon l'une des revendications 1 à 2, dans lequel la position cible (Y) est choisie par un utilisateur.

4. Procédé selon l'une des revendications 1 à 2, comprenant la présélection automatique d'un ensemble de positions relatives de la mandibule par rapport au maxillaire, ledit ensemble de positions étant choisi de sorte que chaque position dudit ensemble définisse une dimension verticale d'occlusion dans une gamme déterminée.

5. Procédé selon la revendication 4, dans lequel la présélection est réalisée à partir de critères cliniques préalablement fournis.

6. Procédé selon l'une des revendications 1 à 3, dans lequel la position cible est choisie de sorte à augmenter une dimension verticale d'occlusion (DVO) d'une valeur inférieure à un seuil choisi pour ne pas provoquer de déplacement de l'axe de rotation des condyles mandibulaires par rapport à la fosse temporale du patient.

7. Procédé selon l'une des revendications 1 à 3, dans lequel la position cible (Y) est choisie de sorte à augmenter une dimension verticale d'occlusion définie au niveau du dentalé de moins de 20 mm par rapport à la dimension verticale d'occlusion définie par la position occlusale de référence (X) du patient.

8. Procédé selon l'une des revendications 1 à 3, dans lequel la position cible est choisie de sorte à diminuer une dimension verticale d'occlusion (DVO).

9. Procédé selon l'une des revendications 1 à 8, comprenant la détermination d'un axe de rotation de la mandibule et la création de positions virtuelles de la mandibule sur le trajet de la rotation dans le sens d'une augmentation ou d'une réduction de la dimension verticale d'occlusion, la position cible étant choisie parmi lesdites positions virtuelles.

10. Procédé selon l'une des revendications 1 à 9, dans lequel, dans ladite animation des modèles des arcades mandibulaire et maxillaire, la position occlusale de référence de la mandibule par rapport au maxillaire dans la relation intermaxillaire corrigée devient la position cible.

11. Procédé selon l'une des revendications 1 à 10, comprenant, à partir de l'enregistrement de la cinématique mandibulaire, le tracé d'un motif de mastication et la comparaison dudit motif avec un motif type fonctionnel.

12. Procédé selon l'une des revendications 1 à 11, dans lequel la position de référence est la position occlusale de référence du patient avant la correction de la relation intermaxillaire.

## Patentansprüche

1. Verfahren zum Animieren von Modellen von Unterkiefer und Oberkiefer eines Patienten in einer korrigierten intermaxillären Beziehung, wobei das Verfahren durch Computer durchgeführt wird und umfasst:
- das Empfangen von dreidimensionalen digitalen Modellen (MD, MX) von Unterkiefer und Oberkiefer des Patienten,
- das Empfangen einer Aufzeichnung der Kieferkinematik (C1) des Patienten, wobei die Aufzeichnung eine Vielzahl relativer Positionen des Unterkiefers des Patienten im Verhältnis zum Oberkiefer umfasst,
- das Auswählen einer Referenzposition (X) des Unterkiefers im Verhältnis zum Oberkiefer des Patienten aus den Positionen, und
- das Bestimmen einer Zielposition (Y) des Unterkiefers im Verhältnis zum Oberkiefer, wobei die Zielposition eine korrigierte intermaxilläre Beziehung des Patienten definiert,
- das Bestimmen einer festen Transformation zwischen der Referenzposition (X) und der Zielposition (Y),
- das Anwenden der festen Transformation (i) auf die Aufzeichnung der Kieferkinematik des Patienten, um die dreidimensionalen digitalen Modelle von Unterkiefer und Oberkiefer zu animieren, oder (ii) auf das dreidimensionale digitale Modell des Unterkiefers, wobei die Aufzeichnung der bereitgestellten Kieferkinematik auf die dreidimensionalen digitalen Modelle des Unterkiefers und des Oberkiefers angewendet wird, um die Modelle zu animieren.

2. Verfahren nach Anspruch 1, wobei die Aufzeichnung der Kieferkinematik des Patienten die Bahn von Markern beschreibt, die mit dem Unterkiefer fest verbunden sind, und die feste Transformation eine Transformation ist, die erlaubt, aus der Position der Marker in der Referenzposition (X) in die Position der Marker in der Zielposition (Y) zu wechseln.

3. Verfahren nach einem der Ansprüche 1 bis 2, wobei die Zielposition (Y) von einem Benutzer gewählt wird.

4. Verfahren nach einem der Ansprüche 1 bis 2, umfassend die automatische Vorauswahl einer Gesamtheit von relativen Positionen des Unterkiefers im Verhältnis zum Oberkiefer, wobei die Gesamtheit von Positionen derart gewählt wird, dass jede Position der Gesamtheit eine vertikale Okklusionsdimension in einem bestimmten Bereich definiert.

5. Verfahren nach Anspruch 4, wobei die Vorauswahl auf der Basis zuvor bereitgestellter klinischer Kriterien durchgeführt wird.

6. Verfahren nach einem der Ansprüche 1 bis 3, wobei die Zielposition derart gewählt ist, dass eine vertikale Okklusionsdimension (DVO) um einen Wert unterhalb eines gewählten Grenzwerts vergrößert wird, um keine Verlagerung der Rotationsachse der Unterkiefergelenke im Verhältnis zum Temporalfenster des Patienten zu bewirken.

7. Verfahren nach einem der Ansprüche 1 bis 3, wobei die Zielposition (Y) derart gewählt ist, dass eine im Bereich des Inzisalpunkts definierte vertikale Okklusionsdimension um weniger als 20 mm im Verhältnis zu der von der okklusalen Referenzposition (X) des Patienten definierten vertikalen Okklusionsdimension vergrößert wird.

8. Verfahren nach einem der Ansprüche 1 bis 3, wobei die Zielposition derart gewählt ist, dass eine vertikale Okklusionsdimension (DVO) verkleinert wird.

9. Verfahren nach einem der Ansprüche 1 bis 8, umfassend das Bestimmen einer Rotationsachse des Unterkiefers und das Erzeugen virtueller Positionen des Unterkiefers auf der Bahn der Rotation im Hinblick auf eine Vergrößerung oder eine Verkleinerung der vertikalen Okklusionsdimension, wobei die Zielposition aus den virtuellen Positionen gewählt ist.

10. Verfahren nach einem der Ansprüche 1 bis 9, wobei in der Animation der Modelle von Unterkiefer und Oberkiefer die okklusale Referenzposition des Unterkiefers im Verhältnis zum Oberkiefer in der korrigierten intermaxillären Beziehung die Zielposition wird.

11. Verfahren nach einem der Ansprüche 1 bis 10, umfassend auf der Basis der Aufzeichnung der Kieferkinematik den Verlauf eines Kaumotivs und den Vergleich des Motivs mit einem typischen Funktionsmotiv.

12. Verfahren nach einem der Ansprüche 1 bis 11, wobei die Referenzposition die okklusale Referenzposition des Patienten vor der Korrektur der intermaxillären Beziehung ist.

## Claims

1. A method for animating models of a patient's mandibular and maxillary arches in a corrected intermaxillary relationship, said method being computer-implemented and comprising:
- receiving three-dimensional digital models (MD, MX) of the patient's mandibular and maxillary arches,
- receiving a record of the patient's mandibular kinematics (C1), said record comprising a plurality of relative positions of the patient's mandible relative to to the maxilla,
- selecting, from said positions, a reference position (X) of the mandible relative to the patient's maxilla, and
- determining a target position (Y) of the mandible relative to the maxilla, said target position defining the patient's corrected intermaxillary relationship,
- determining a rigid transformation between the reference position (X) and the target position (Y),
- applying said rigid transformation to (i) the record of the patient's mandibular kinematics to animate the three-dimensional digital models of the mandibular and maxillary arches, or (ii) the three-dimensional digital model of the mandible, the record of the mandibular kinematics provided being applied to the three-dimensional digital models of the mandible and maxilla to animate said models.

2. The method according to claim 1, wherein the record of the patient's mandibular kinematics describes the trajectory of markers integral with the mandible, and the rigid transformation is a transformation for switching from the position of the markers in the reference position (X) to the position of the markers in the target position (Y).

3. The method according to one of claims 1 to 2, wherein the target position (Y) is chosen by a user.

4. The method according to one of claims 1 to 2, comprising automatically preselecting a set of relative positions of the mandible relative to the maxilla, said set of positions being chosen such that each position of said set defines a vertical dimension of occlusion within a determined range.

5. The method according to claim 4, wherein the preselection is made from previously provided clinical criteria.

6. The method according to one of claims 1 to 3, wherein the target position is chosen so as to increase a vertical dimension of occlusion (VDO) by a value less than a threshold chosen so as not to cause movement of the axis of rotation of the mandibular condyles relative to the patient's temporal fossa.

7. The method according to one of claims 1 to 3, wherein the target position (Y) is chosen so as to increase a vertical dimension of occlusion defined at the incisor crown end by less than 20 mm relative to the vertical dimension of occlusion defined by the patient's reference occlusal position (X).

8. The method according to one of claims 1 to 3, wherein the target position is chosen so as to decrease a vertical dimension of occlusion (VDO).

9. The method according to one of claims 1 to 8, comprising determining an axis of rotation of the mandible and creating virtual positions of the mandible in the path of the rotation towards increasing or decreasing the vertical dimension of occlusion, the target position being chosen from said virtual positions.

10. The method according to one of claims 1 to 9, wherein in said animating the models of the mandibular and maxillary arches, the reference occlusal position of the mandible relative to the maxilla in the corrected intermaxillary relationship becomes the target position.

11. The method according to one of claims 1 to 10, comprising, from the record of the mandibular kinematics, tracing a mastication pattern and comparing said pattern with a typical functional pattern.

12. The method according to one of claims 1 to 11, wherein the reference position is the patient's reference occlusal position prior to correcting the intermaxillary relationship.
